# FASCICULE DE BREVET EUROPEEN

(11) **EP 2 593 543 B1**
(45) Date de publication et mention de la délivrance du brevet: **22.02.2017**
(21) Numéro de dépôt: 11740955.7
(22) Date de dépôt: 16.06.2011
(51) Int. Cl.: C12N 9/24, C12N 9/42, C12P 1/02, C12P 7/10, C12P 19/00, C12R 1/885

(54) **PROCÉDÉ DE PRODUCTION D'ENZYMES CELLULOLYTIQUES ET/OU HÉMICELLULOLYTIQUES AMÉLIORÉ**
VERBESSERTES VERFAHREN ZUR HERSTELLUNG VON CELLULOLYTISCHEN UND/ODER HEMICELLULOLYTISCHEN ENZYMEN
AMELIORATED PROCESS FOR THE PRODUCTION OF CELLULOLYTIC AND/OR HEMICELLULOLYTIC ENZYMES

(30) Priorité: 12.07.2010 FR 1002923
(43) Date de publication de la demande: 22.05.2013
(73) Titulaire: IFP Énergies nouvelles, 92852 Rueil-Malmaison Cedex (FR)
(72) Inventeur: BEN CHAABANE, Fadhel, F-75020 Paris (FR); MONOT, Frédéric, F-92000 Nanterre (FR)
(86) Numéro de dépôt international: PCT/FR2011/000350
(87) Numéro de publication internationale: WO 2012/007650

(56) Documents cités:
- EP-A1- 1 690 944
- POURQUIÉ J ET AL: "Scale up of cellulase production and utilization" In: "BIOCHEMISTRY AND GENETICS OF CELLULOSE DEGRADATION", 1 janvier 1988 (1988-01-01), FEMS SYMPOSIUM, ACADEMIC PRESS, LONDON, GB, PAGE(S) 71 - 86, XP009141720, ISSN: 0163-9188 page 72 - page 79; figure 1; tableau 6
- HERPOËL-GIMBERT ISABELLE ET AL: "Comparative secretome analyses of two Trichoderma reesei RUT-C30 and CL847 hypersecretory strains.", BIOTECHNOLOGY FOR BIOFUELS 2008 LNKD- PUBMED:19105830, vol. 1, no. 1, 23 décembre 2008 (2008-12-23), pages 18-29, XP002612023, ISSN: 1754-6834
- CHAUVE MARIE ET AL: "Comparative kinetic analysis of two fungal beta-glucosidases.", BIOTECHNOLOGY FOR BIOFUELS 2010 LNKD- PUBMED:20181208, vol. 3, no. 1, 11 février 2010 (2010-02-11), pages 3-10, XP002612024, ISSN: 1754-6834
- POURQUIÉ J ET AL: "Cellulase production by Trichoderma reesei", BIOCONVERSION OF FOREST AND AGRICULTURAL PLANT RESIDUES, CAB INTERNATIONAL, GB, 1 janvier 1993 (1993-01-01), pages 107-116, XP009141741, ISBN: 978-0-85198-798-9
- LING MIN ET AL: "Induction of cellulase gene transcription by a novel oligosaccharide: molasses alcohol stillage substance", WORLD JOURNAL OF MICROBIOLOGY & BIOTECHNOLOGY, vol. 25, no. 8, août 2009 (2009-08), pages 1485-1489, XP002612025, ISSN: 0959-3993

## Description

### DOMAINE DE L'INVENTION

L'invention est relative à un procédé de production d'enzymes pour l'hydrolyse de biomasse lignocellulosique.

### ART ANTÉRIEUR

L'augmentation des capacités de production de bioéthanol pour ses qualités de biocarburant est aujourd'hui un sujet d'actualité. Les objectifs d'incorporation sont en cours de discussion au sein de l'Union Européenne sur la base d'une proposition initiale de 20% d'utilisation d'énergies renouvelables d'ici 2020 avec une incorporation de 10% de biocarburants selon des critères de durabilité qui devraient favoriser les biocarburants de seconde génération produits à partir de biomasse lignocellulosique.

La biomasse lignocellulosique se caractérise par une structure complexe constituée de trois principaux polymères : la cellulose, l'hémicellulose et la lignine.

De façon classique, le procédé de transformation de biomasse en éthanol comprend plusieurs étapes. Un prétraitement permet de rendre la cellulose et éventuellement les hémicelluloses qui sont les cibles de l'hydrolyse enzymatique accessibles aux enzymes. Le prétraitement vise à modifier les propriétés physiques et physico-chimiques du matériau lignocellulosique, en vue d'améliorer l'accessibilité de la cellulose emprisonnée dans la matrice de lignine et d'hémicellulose. L'étape d'hydrolyse enzymatique permet la transformation de la cellulose et des hémicelluloses en sucres par utilisation d'enzymes cellulolytiques et/ou hémicellulolytiques.

Les sucres obtenus par hydrolyse de la biomasse lignocellulosique sont des pentoses (xylose et arabinose principalement), des disaccharides (cellobiose) et du glucose qui peuvent être fermentés par les microorganismes. Le glucose peut, par exemple, être facilement transformé en éthanol par la levure *Saccharomyces cerevisiae* lors de l'étape de fermentation alcoolique.

Enfin, une étape de distillation permet de séparer et de récupérer le produit issu de la fermentation ainsi obtenu, donc l'éthanol dans le cas précédent, du moût de fermentation.

Les différentes études technico-économiques démontrent la nécessité de réduire le coût liés à l'étape d'hydrolyse enzymatique pour amener le coût de l'éthanol produit à des valeurs proches de l'éthanol obtenu à partir d'amidon.

A l'heure actuelle, les cellulases industrielles sont principalement produites par un champignon filamenteux, *Trichoderma reesei,* en raison de son fort pouvoir de sécrétion des cellulases.

Un des moyens de diminution des coûts consiste à optimiser les conditions opératoires du procédé de production de cellulases de façon à augmenter la productivité ou à obtenir un cocktail enzymatique ayant une activité spécifique améliorée.

Les souches sauvages de *Trichoderma reesei* ont la faculté de secréter, en présence d'un substrat inducteur, un complexe enzymatique bien adapté à l'hydrolyse de la cellulose. Les enzymes du complexe enzymatique contiennent trois grands types d'activité : les endoglucanases, les exoglucanases et les cellobiases. D'autres protéines, telles que les xylanases, nécessaires pour l'hydrolyse de la biomasse lignocellulosique sont également produites par *Trichoderma reesei.* La présence d'un substrat inducteur est indispensable à l'expression des enzymes cellulolytiques et/ou hémicellulolytiques.

La régulation des gènes de cellulases sur différentes sources de carbone a été étudiée en détail. Le glucose exerce une répression catabolique sur la production de cellulases. Celle-ci est induite en présence de cellulose, de ses produits d'hydrolyse comme le cellobiose ou de certains oligosaccharides, notamment des disaccharides comme le lactose ou le sophorose (Ilmén et al. 1997, Appl. Environ. Microbiol. Vol 63, p1298-1306). La nature du substrat carboné a une forte influence sur la composition du complexe enzymatique. Ainsi, le xylose, associé à un substrat carboné inducteur comme la cellulose ou le lactose permet d'améliorer significativement l'activité dite xylanase lorsqu'il est présent à des concentrations limitantes de l'ordre de 0,5 à 1 mM (Mach-Aigner et al., 2010 Applied and Environmental Microbiology, Vol76 N°6, p1770-1776 ). La solubilisation des hémicelluloses résiduelles (les xylanes) par les xylanases permettraient de favoriser l'hydrolyse enzymatique (Vàrnai et al., 2010 Enzyme and Microbial Technology Vol 46 p185-193).

Pour obtenir de bonnes productivités en enzymes, il est nécessaire d'apporter une source de carbone rapidement assimilable pour permettre une croissance rapide de *Trichoderma reesei,* et un substrat inducteur qui permette l'expression des cellulases et la sécrétion dans le milieu de culture. La cellulose peut jouer ces deux rôles. Cependant, elle est difficile d'utilisation au stade industriel et elle a été remplacée par des sources de carbones solubles telles que le lactose jouant également le rôle de substrat inducteur.

D'autres sucres tels que le cellobiose ou le sophorose ont également été décrits comme inducteurs (Ilmen et al. (1997), Foreman et al. (2003) Biol Chem 278, p31988-31997, Pakula et al. (2005) Microbiology, 151, p135-143) mais sont trop onéreux pour être utilisés au stade industriel.

De façon générale, il a été constaté que les productions de cellulases par *Trichoderma reesei* avec des substrats solubles sont très inférieures à celles obtenues sur cellulose en mode « batch » (c'est à dire en milieu fermé) en raison de l'effet répresseur des sucres facilement assimilables à forte concentration.

Le brevet FR-B-2 555 603, déposé au nom de la Demanderesse, propose une alimentation en continu des substrats carbonés permettant de lever la répression catabolique en limitant la concentration résiduelle dans les cultures et en optimisant la quantité de sucres, pour obtenir un meilleur rendement et une meilleure productivité enzymatique. Le procédé décrit dans ce brevet propose de démarrer l'alimentation en substrat en mettant en oeuvre des sucres solubles comme source de carbone, éventuellement sous forme de mélange. Toutefois un apport continu limitant de glucose ou de xylose non associé à du lactose ou à un autre inducteur de cellulases (sophorose, cellobiose,...) ne permet pas d'obtenir de fortes productivités en enzymes.

Le lactose reste, dans les procédés de production industriels d'enzymes cellulolytiques, un des substrats les plus appropriés. En plus d'être élevé, son prix est très fluctuant et il représente environ un tiers du prix de revient des enzymes.

Une des solutions envisagées et présentée dans le brevet EP-B-0 448 430 consiste à utiliser des substrats carbonés issus de la filière, par exemple les hémicelluloses hydrolysées, comme source de carbone inductrice. Toutefois, la productivité reste inférieure d'environ 50% par rapport au procédé utilisant le lactose seul comme substrat inducteur.

La demande de brevet WO 2009/026716 décrit un procédé de production de cellulases dans lequel les dérivés hémicellulolytiques représentent plus de 40% de la source de carbone et les sucres inducteurs de la production de cellulases représentent entre 3 et 20 % de ce mélange. Ce procédé permet de produire au moins deux fois plus de cellulases par rapport à un procédé utilisant uniquement des sucres issus des hémicelluloses. Toutefois, les performances de production de cellulases décrites restent encore inférieures à un procédé utilisant uniquement du lactose.

Les recherches actuelles montrent qu'il est nécessaire, pour améliorer les procédés de production d'enzymes cellulolytiques et/ou hémicellulolytiques de développer de nouveaux substrats inducteurs, au moins aussi performants que ceux utilisant le lactose et à moindre coût.

C'est dans ce cadre que s'inscrit la présente invention.

### RÉSUMÉ DE L'INVENTION

La présente invention décrit un procédé de production d'enzymes cellulolytiques et/ou hémicellulolytiques dans lequel le substrat inducteur est un mélange de glucose, lactose et xylose.

### DESCRIPTION DÉTAILLÉE DE L'INVENTION

Le procédé de production d'enzymes cellulolytiques et/ou hémicellulolytiques par un microorganisme cellulolytique et/ou hémicellulolytique selon la présente invention comprend au moins une phase de croissance en présence d'une source de carbone et au moins une phase de production en présence d'un substrat inducteur, dans lequel ledit substrat inducteur est un mélange de glucose ou hydrolysats cellulosiques, de lactose et de xylose ou d'une solution d'hydrolysats hémicellulolytiques, les teneurs de chacun des constituants du mélange étant définies par les limites suivantes :
- de 40 à 65 % poids de glucose,
- de 21 à 25 % poids de lactose et
- de 10 à 39% poids de xylose,
la somme de ces trois constituants étant égale à 100%.

Le substrat inducteur est dépourvu de tout autre sucre que les constituants listés ci-dessus. Ainsi, les proportions relatives de chacun des constituants sont choisies de sorte que la somme des teneurs pondérales est égale à 100%.

Grâce au procédé selon l'invention mettant en oeuvre un mélange de sucres particuliers comme substrat inducteur, les concentrations finales en protéines obtenues sont de 50 à 60% supérieures à celles obtenues avec le lactose utilisé comme unique substrat de production. Elles sont également environ 3 fois supérieures à celle obtenue avec des mélanges améliorés riches en xyloses tels que décrits dans l'art antérieur WO 2009/026716.

Le procédé selon la présente invention utilise un mélange à base de glucose. Plus de 60% du lactose, inducteur de la production de cellulase peut être remplacé par du glucose, connu portant pour être répresseur de la production de cellulases, avec une performance finale du mélange inducteur améliorée. Le glucose présente un coût beaucoup plus faible que le lactose et une solubilité dans l'eau environ 3 fois plus élevée. Il est donc possible de diminuer les volumes mis en jeu. Ainsi, dans ce procédé, le glucose est à la fois utilisé dans la phase de croissance mais également dans la phase de production à hauteur de 60% du mélange constituant le substrat inducteur.

Le glucose utilisé dans le mélange de sucres peut également être remplacé par du glucose issu de l'étape d'hydrolyse enzymatique de la cellulose, donc directement issu du procédé de transformation de biomasse lignocellulosique en éthanol. Cela contribue à diminuer le coût de la production d'enzymes par l'utilisation de co-produits du procédé. On parle alors d'hydrolysats cellulosiques.

Le xylose utilisé dans le mélange constituant le sucre inducteur peut être remplacé par une solution d'hydrolysats hémicellulolytiques, issue du procédé de transformation de biomasse lignocellulosique en éthanol et notamment issue du prétraitement de la biomasse.

D'autre part, la possibilité d'utiliser un mélange de très concentré en sucres permet avantageusement de limiter les risques de contaminations.

Le fait d'ajouter dans le mélange constituant le substrat inducteur du xylose permet d'augmenter fortement l'activité xylanase du cocktail enzymatique final. Le xylose peut être avantageusement remplacé par une solution d'hydrolysat hémicellulosique, issue de l'hydrolyse des hémicelluloses lors du prétraitement de la biomasse lignocellulosique dans les procédés de production de biocarburants de deuxième génération, et qui peut être concentrée si nécessaire.

De façon préférée, le mélange constituant le substrat inducteur comprend 50 à 65 % poids de glucose, 22 à 24 % poids de lactose et de 15 à 25% poids de xylose, éventuellement issue d'un prétraitement de la biomasse lignocellulosique, la somme des constituants étant égale à 100%.

Très préférentiellement, le substrat inducteur est un mélange constitué de 60 % poids de glucose, 23% poids de lactose et 17% poids de xylose .

Le substrat carboné utilisé pendant la phase de croissance est choisi parmi le glucose, le xylose, le lactose, les résidus obtenus après fermentation éthanolique des sucres monomères des hydrolysats enzymatiques de biomasse cellulosique et/ou un extrait brut de pentoses hydrosolubles provenant éventuellement du prétraitement d'une biomasse cellulosique.

Très préférentiellement, le substrat de croissance est le glucose.

Les souches industrielles utilisées appartiennent à l'espèce *Trichoderma reesei,* modifiées pour améliorer les enzymes cellulolytiques et/ou hémicellulolytiques par les processus de mutation-sélection. On citera par exemple la souche IFP CL847. Des souches améliorées par des techniques de recombinaison génétiques peuvent aussi être utilisées. Elles doivent être délétées pour la répression catabolique par le glucose (Δ CRE1), comme par exemple CL847.

Ces souches sont cultivées en bioréacteurs agités et aérés dans des conditions compatibles avec leur croissance et la production des enzymes. Les conditions sont telles que le pH est ajusté entre 3.5 et 6 et la température entre 20 et 35 °C. On choisira de préférence un pH de 4,8 et une température de 27°C durant la phase de croissance et un pH de 4 et une température de 25°C durant la phase de production. Le taux d'aération exprimé en volume d'air par volume de milieu réactionnel et par minute ou vvm appliqué durant le procédé est comprise entre 0.3 et 1.5 min-1 et la vitesse de rotation ou rpm doit permettre de réguler la pression en 02 entre 20% et 60%. On choisira de préférence une aération de 0.5 vvm et une agitation permettant de réguler la pression en 02 à 30 %.

Selon sa nature, le substrat carboné choisi pour l'obtention de la biomasse est introduit dans le bioréacteur avant stérilisation, ou est stérilisé séparément et est introduit dans le bioréacteur après stérilisation de ce dernier pour avoir une concentration initiale en sucres de 15 à 60 g/L.

En vue de la phase de production, une solution aqueuse contenant le substrat inducteur constitué du mélange glucose/lactose/xylose ou solution d'hydrolysat hémicellulosique choisi pour la phase de production des enzymes est préparée à une concentration de 350 à 600 g/L dans la solution d'alimentation utilisée.
Préférentiellement, la concentration est comprise entre 450 et 550 g/L.

La solution aqueuse est injectée après l'épuisement du substrat initial de façon à apporter une quantité optimisée. Le flux d'introduction de la solution d'alimentation est de 30 à 45 mg par gramme de cellules et par heure.

La concentration résiduelle en sucre dans le milieu de culture est inférieure à 1 g/L pendant la phase de production ("fed-batch") de façon à limiter la production de biomasse. De façon préférée, cette concentration est inférieure à 0,5 g/l et encore plus préférentiellement à 0,1 g/L

### EXEMPLES

Parmi les exemples qui suivent, l'exemple 1 présente une culture utilisant le glucose comme substrat carboné pendant la phase de croissance et la phase de production. Cet exemple démontre la faible production de protéines obtenue lorsque ce sucre est utilisé comme unique substrat carboné même si le flux est limitant durant la phase fed-batch (concentration résiduelle en glucose proche de 0). Le deuxième exemple représente la fermentation de référence utilisant le lactose comme substrat carboné pendant la phase de croissance et la phase de production qui permet une forte production de protéines. L'exemple 3 reproduit une expérience utilisant en phase de production un mélange tel que décrit dans la demande de brevet WO 2009/026716 contenant une forte proportion en xylose permettant selon les auteurs d'augmenter fortement la production des cellulases par rapport à une expérience où le xylose ou les dérivés hémicellulosiques sont utilisés comme uniques substrats carbonés. Les exemples 4 à 6 sont ceux du brevet conformes au procédé selon la présente invention.

### Exemple 1 : Production d'enzymes sur glucose (non conforme à l'invention)

La production de cellulases est effectuée en bioréacteur agité mécaniquement. Le milieu minéral a la composition suivante : KOH 1,66 g./L, H3PO4 85 % 2 mL/L, (NH4)2SO4 2,8 g/L, MgSO4, 7 H2O 0,6 g/L, CaCL2 0,6 g/L, MnSO4 3,2 mg/L, ZnSO4, 7 H2O 2,8 mg/L, CoCl2 10 4,0 mg/L, FeSO4, 7 H2O 10 mg/L, Corn Steep 1,2 g/L, anti-mousse 0,5 mL/L. Le bioréacteur contenant le milieu minéral est stérilisé à 120 °C pendant 20 minutes , la source carbonée glucose est stérilisée à part à 120°C pendant 20 minutes puis ajoutée stérilement dans le bioréacteur de façon à avoir une concentration finale de 30 g/L. Le bioréacteur est ensemencé à 10% (v/v) avec une préculture liquide de la souche de *Trichoderma reesei* CL847. Le milieu minéral de la préculture, est identique à celui du bioréacteur mis à part l'addition de phtalate de potassium à 5 g.L-1 pour tamponner le pH. La croissance du champignon en préculture est faite en utilisant le glucose comme substrat carboné, à la concentration de 30 g/L. La croissance de l'inoculum dure de 2 à 3 jours et est effectuée à 28 °C dans un incubateur agité. Le transfert vers le bioréacteur est réalisé si la concentration résiduelle en glucose est inférieure à 15 g/L

L'expérience effectuée en bioréacteur comporte deux phases :
- Une phase de croissance sur substrat carboné glucose (concentration initiale = 30 g/L) à une température de 27 °C et un pH de 4,8 (régulé par de l'ammoniaque 5.5 M). L'aération est de 0,5 vvm et l'agitation est augmentée entre 200 et 800 rpm en fonction de la pO2 (pression en oxygène dissous), qui est maintenue supérieure à 30 %.
- Une phase de production d'enzymes. Lorsque le substrat initial du fermenteur est épuisé et la solution de glucose à 250 g/L est injectée en continu au débit de 30 à 40 mg par g de cellules et par heure jusqu'à 164 heures. La température est baissé à 25 °C et le pH à 4 jusqu'à la fin de la culture. Le pH est régulé par addition d'une solution d'ammoniaque à 5.5 N qui apporte l'azote nécessaire à la synthèse des protéines excrétées. La teneur en oxygène dissous est maintenue au-dessus de 15 à 20 % par action sur l'aération et l'agitation.

La production d'enzymes est suivie par le dosage des protéines extracellulaires par la méthode de Lowry et standard BSA, après séparation du mycélium par filtration ou centrifugation. Les activités cellulolytiques déterminées sont:
- L'activité papier filtre (UPF : unité papier filtre) qui permet de doser l'activité globale du pool enzymatique endoglucanases et exoglucanases
- Les activités aryl β-glucosidase et xylanases pour les activités spécifiques.

L'activité UPF est mesurée sur papier Whatman n° 1 (Procédure recommandée par la commission biotechnologique IUPAC) à la concentration initiale de 50 g/L ; on détermine la prise d'essai de la solution enzymatique à analyser qui libère l'équivalent de 2 g/L de glucose (dosage colorimétrique) en 60 minutes. Le principe de l'activité papier filtre est de déterminer par dosage au DNS (acide dinitrosalicylique) la quantité de sucres réduits issue d'un papier Whatman N°1 (Procédure recommandée par la commission biotechnologique IUPAC).
Le substrat utilisé pour déterminer l'activité aryl β-glucosidase est le p-nitrophenyl-ß-D-glucopyranoside (PNPG). Il est clivé par la ß-glucosidase qui libère le p-nitrophenol.

Une unité d'activité aryl ß-glucosidase est définie comme la quantité d'enzyme nécessaire pour produire 1µmol de p-nitrophenol à partir de PNPG par minute et est exprimé en IU/mL.
Le principe du dosage de l'activité xylanase repose sur la détermination par dosage DNS de la quantité de sucres réduits issue de la solution xylanase hydrolysée. Cette méthode de dosage utilise les propriétés réductrices des sucres et principalement du xylose. L'activité xylanase est exprimée en IU/mL correspond à la quantité d'enzyme nécessaire pour produire 1µmol de xylose par minute.
Les activités spécifiques sont obtenues en divisant les activités exprimées en IU/mL par la concentration en protéines. Elles sont exprimées en IU/mg.

Les déterminations analytiques sur le moût final de l'exemple 1 donnent les résultats suivants :
Les déterminations analytiques sur le moût final donnent les résultats suivants :
   Biomasse g/L 15.2
   Protéines g/L 2,9
   UPF IU/mL 1,4
   Xylanase 29.1 IU/mg
   β-Glucosidase spécifique 0.35 IU/mg

La souche CL847 est présentée comme étant déréprimée à la répression catabolique exercée par le glucose sur la production de cellulase (elle est délétée du gène CRE1). Il apparaît que même pour un fed-batch conduit dans des conditions de limitation en glucose, la production finale en protéines est très faible.

### Exemple 2 : Production d'enzymes sur lactose (non conforme à l'invention)

La production d'enzymes est menée dans les mêmes conditions que dans l'Exemple 1. Le substrat carboné pendant les phases de croissance et de production est le lactose pur. Le lactose est un inducteur important de la production de cellulases. C'est le substrat qui est le plus utilisé industriellement pour la production de cellulases.
Après 30 heures de croissance, après l'épuisement du substrat initial, la solution de fed-batch à 250 g/L est injectée en continu au débit de 35 mg par g de cellules et par heure jusqu'à 164 heures.

Les déterminations analytiques sur le moût final donnent les résultats suivants :
Biomasse g/L 13.5
Protéines g/L 37.8
UPF IU/mL 22.1
Xylanase 408.5 IU/mL
β-Glucosidase spécifique 0.96 IU/mg

### Exemple 3: Production avec le mélange 97% xylose / 3% de sucres inducteurs de la production de cellulases ou CIC (non conforme à l'invention)

L'expérience a été menée dans les mêmes conditions que l'exemple 1 en utilisant le xylose pur comme unique substrat carboné au cours de la phase de croissance et en phase de production le mélange 97% xylose / 3% CIC présenté dans la demande de brevet WO 2009/026716 A1. Les CIC (sucres inducteurs de la production de cellulases) sont un mélange de sucres permettant l'induction de la production des cellulases. Sa composition est la suivante: 56% gentiobiose, 14% sophorose, 10% trehalose, 6% cellobiose, 6% maltotriose, 8%glucose.
Après épuisement du substrat initial, le mélange 97% xylose / 3%CIC à 360 g/L est injecté selon les recommandations décrites au débit de 0.4 g de carbone .L-1.h-1

Les déterminations analytiques sur le moût final donnent les résultats suivants :
Biomasse 14.3 g/L
Protéines 18.7 g/L
UPF 6.4 IU/mL
Xylanase 6000.1 IU/mL
UPF spécifique 0.34 IU/mg
β-Glucosidase spécifique 1,15 IU/mg

La production finale en protéines est proche de celle présentée dans le brevet WO 2009/026716 A1 où les auteurs ont obtenu une concentration finale de protéines de 25 g/L avec la souche P59G. L'activité spécifique FPase (0.34IU/mg) est faible alors que l'activité xylanase est très élevée.

### Exemple 4: Production avec le mélange Lactose (23 %)/Glucose (60 %)/Xylose (17 %) à 500 g/L (selon l'invention)

L'expérience de l'exemple 4 est menée dans les mêmes conditions que l'exemple 1 avec le glucose comme substrat carboné de croissance à 60 g/L. Nous avons ensuite utilisé au cours de la phase de production en mode "fed-batch" une solution où trois substrats carbonés sont mélangés selon les proportions suivantes : Lactose (23 %)/Glucose (60 %)/Xylose (17 %) à 500 g/L. Ce mélange a été injecté à 45 mg.g⁻¹.h⁻¹. Les déterminations analytiques sur le moût final donnent les résultats suivants :
Biomasse 26.1 g/L
Protéines 61.8 g/L
UPF 33.7 IU/mL
Xylanase 4017 IU/mL
UPF spécifique 0.55 IU/mg
β-Glucosidase spécifique 1,2 IU/mg

La production finale en protéines est plus de 3 fois supérieure à celle de l'exemple 3. L'activité papier filtre qui est indicatrice de l'activité cellulase est environ 5 fois supérieure. L'activité xylanase est 10 fois supérieure à celle de l'exemple 2 réalisée avec le lactose comme unique substrat carboné de croissance et de production mais environ 50% inférieure à celle de l'exemple 3.

### Exemple 5: Utilisation du mélange Lactose (23 %)/Glucose (60 %)/solution de sucres en C5 (17 %) à 500 g/L (selon l'invention)

L'exemple 5 est conduit dans les mêmes conditions que l'exemple 4. Le glucose est utilisé durant la phase de croissance à une concentration de 15 g/L. Le xylose de la solution de fed-batch est remplacé par une solution hémicellulosique soluble ou solution de sucres en C5 issue d'une paille de blé imprégnée de 0,08 N d'acide sulfurique et prétraitée par explosion à la vapeur (19 bar, 5 minutes). Le mélange est injecté à 30 mg.g⁻¹.h⁻¹ pendant 170h.

Les déterminations analytiques sur le moût final donnent les résultats suivants :
Biomasse 19.1 g/L
Protéines 57.3 g/L
UPF 25.1 IU/mL
Xylanase 1151 IU/mL
UPF spécifique 0.44 IU/mg
β-Glucosidase spécifique 1,4 IU/mg

Cette expérience a permis d'augmenter les productions en protéines de plus de 50% par rapport à l'exemple 2 et l'activité xylanase est presque 3 fois supérieure. La majorité du lactose est remplacée durant la phase de croissance et de production par du glucose et de la solution de sucres en C5. La concentration du glucose durant la phase de croissance est diminuée par rapport à l'exemple 4, de 60 à 15 g/L . La concentration en biomasse est diminuée de 26 à 19 g/L mais cela a peu d'incidence sur la concentration finale en protéines (baisse de 7%). On a donc ainsi une utilisation optimale des sucres et une diminution notable du coût de la source de carbone.

### Exemple 6: Utilisation du mélange : Lactose (23%)/Hydrolysats C6 (56%)/xylose (21%) à 500 g/L (selon l'invention)

L'exemple 6 est conduit dans les mêmes conditions que l'exemple 4. Le glucose est utilisé durant la phase de croissance à une concentration de 15 g/L. Le glucose de la solution de fed-batch est remplacé par une solution d'hydrolysat issue de l'hydrolyse enzymatique d'une paille de blé blé imprégnée de 0,08 N d'acide sulfurique et prétraitée par explosion à la vapeur (19 bar, 5 minutes). L'hydrolyse enzymatique de la paille de blé a été conduite à 50°C et à pH 4.8. Elle a aboutit à une hydrolyse de 95% de la cellulose en glucose.
Du lactose et du xylose ont été dissouts dans cette solution de façon à aboutir au mélange : Lactose (23%)/Hydrolysats C6 (56%)/xylose (21%) à 500 g/L
Le mélange est injecté à 30 mg.g⁻¹.h-1 pendant 170h durant la phase fed-batch.
Les déterminations analytiques sur le moût final donnent les résultats suivants :
Biomasse 16.1 g/L
Protéines 43.1 g/L
UPF 31.4 IU/mL
Xylanase 6595.1 IU/mL
UPF spécifique 0.73 IU/mg
β-Glucosidase spécifique 1,2 IU/mg

Le cocktail enzymatique obtenu est de très bonne qualité puisque l'activité FPase finale est proche de l'activité obtenue dans l'exemple 4 même si la concentration en protéines est plus faible. L'activité FPase finale est environ 5 fois supérieure à celle obtenue avec l'exemple 3 et environ 50 % supérieure à celle obtenue lors de l'exemple 2 où le lactose est utilisé comme unique substrat carboné lors de la phase Fed-batch.

## Revendications

1. Procédé de production d'enzymes cellulolytiques et/ou hémicellulolytiques par un microorganisme cellulolytique et/ou hémicellulolytique comprenant au moins une phase de croissance en présence d'une source de carbone et au moins une phase de production en présence d'un substrat inducteur, dans lequel ledit substrat inducteur est un mélange de glucose ou hydrolysats cellulosiques, de lactose et de xylose ou d'une solution d'hydrolysats hémicellulolytiques, les teneurs de chacun des constituants du mélange étant définies par les limites suivantes :
- de 40 à 65 % poids de glucose,
- de 21 à 25 % poids de lactose et
- de 10 à 39% poids de xylose,
la somme de ces trois constituants étant égale à 100%, et
le microorganisme appartient à l'espèce Trichoderma reesei, et est délété pour la répression catabolique par le glucose.

2. Procédé selon la revendication 1 dans lequel l'apport de substrat inducteur est effectué en solution, la concentration en substrat inducteur dans la solution d'alimentation utilisée pendant la phase de production étant de 350 à 600 g/L.

3. Procédé selon la revendication 2 dans lequel la concentration est comprise entre 450 et 550g/L.

4. Procédé selon l'une des revendications précédentes dans lequel le mélange constituant le substrat inducteur comprend 50 à 65 % poids de glucose, 22 à 24 % poids de lactose et de 15 à 25% poids de xylose, la somme des constituants du mélange étant égale à 100%.

5. Procédé selon la revendication 4 dans lequel le substrat inducteur est un mélange constitué de 60 % poids de glucose , 23% poids de lactose et 17% poids de xylose.

6. Procédé selon l'une des revendications précédentes dans lequel le substrat carboné utilisé pendant la phase de croissance est choisi parmi le glucose, le xylose, le lactose, les résidus obtenus après fermentation éthanolique des sucres monomères des hydrolysats enzymatiques de biomasse cellulosique et/ou un extrait brut de pentoses hydrosolubles provenant éventuellement du prétraitement d'une biomasse cellulosique.

7. Procédé selon l'une des revendications précédentes dans lequel le pH est ajusté entre 3,5 et 6 et la température est comprise entre 20 et 35°C.

8. Procédé selon l'une des revendications précédentes dans lequel le flux d'introduction de la solution d'alimentation est de 30 à 45 mg par gramme de cellules et par heure.

9. Procédé selon l'une des revendications précédentes dans lequel la concentration résiduelle en sucre dans le milieu de culture pendant la phase de production est inférieure à 1 g/L, préférentiellement à 0,5 g/l et très préférentiellement à 0,1 g/L.

## Patentansprüche

1. Verfahren zur Produktion zellulolytischer und/oder hemizellulolytischer Enzyme durch einen zellulolytischen und/oder hemizellulolytischen Mikroorganismus, umfassend mindestens eine Wachstumsphase in Anwesenheit einer Kohlenstoffquelle und mindestens eine Produktionsphase in Anwesenheit eines Induktorsubstrats, wobei das Induktorsubstrat eine Mischung von Glucose oder Cellulosehydrolysaten, Lactose und Xylose oder eine Lösung hemizellulolytischer Hydrolysate ist, wobei die Gehalte jedes der Bestandteile der Mischung durch die folgenden Grenzen definiert werden:
- von 40 bis 60 Gew.-% Glucose,
- von 21 bis 25 Gew.-% Lactose und
- von 10 bis 39 Gew.-% Xylose,
wobei die Summe dieser drei Bestandteile gleich 100 % ist, und
der Mikroorganismus zur Art Trichoderma reesel gehört und für die katabolische Repression durch Glucose deletiert ist.

2. Verfahren nach Anspruch 1, wobei das Einbringen des Induktorsubstrats in Lösung durchgeführt wird, wobei die Konzentration des Induktorsubstrats in der verwendeten Nährlösung während der Produktionsphase von 350 bis 600 g/l beträgt.

3. Verfahren nach Anspruch 2, wobei die Konzentration zwischen 450 und 550 g/l liegt.

4. Verfahren nach einem der vorhergehenden Ansprüche, wobei die Mischung, die das Induktorsubstrat bildet, 50 bis 65 Gew.-% Glucose, 22 bis 24 Gew.-% Lactose und 15 bis 25 Gew.-% Xylose enthält, wobei die Summe der Bestandteile der Mischung gleich 100 % ist.

5. Verfahren nach Anspruch 4, wobei das Induktorsubstrat eine Mischung ist, die aus 60 Gew.-% Glucose, 23 Gew.-% Lactose und 17 Gew.-% Xylose besteht.

6. Verfahren nach einem der vorhergehenden Ansprüche, wobei das kohlenstoffhaltige Substrat, das während der Wachstumsphase verwendet wird, ausgewählt wird aus Glucose, Xylose, Lactose, den Rückständen, die nach ethanolischer Fermentation monomerer Zucker enzymatischer Hydrolysate von Cellulose-Biomasse erhalten werden, und/oder einem Rohextrakt wasserlöslicher Pentosen, die gegebenenfalls von einer Vorbehandlung einer Cellulose-Biomasse stammen.

7. Verfahren nach einem der vorhergehenden Ansprüche, wobei der pH zwischen 3,5 und 6 eingestellt wird, und die Temperatur zwischen 20 und 35°C liegt.

8. Verfahren nach einem der vorhergehenden Ansprüche, wobei der Einführstrom der Nährlösung 30 bis 45 mg pro Gramm Zellen und pro Stunde beträgt.

9. Verfahren nach einem der vorhergehenden Ansprüche, wobei die Restzuckerkonzentration in dem Kulturmedium während der Produktionsphase weniger als 1 g/l, vorzugsweise weniger als 0,5 g/l und sehr bevorzugt weniger als 0,1 g/l beträgt.

## Claims

1. A process for the production of cellulolytic and/or hemicellulolytic enzymes by a cellulolytic and/or hemicellulolytic microorganism, comprising at least one phase for growth in the presence of a source of carbon and at least one phase for production in the presence of an inducing substrate, in which said inducing substrate is a mixture of glucose or cellulosic hydrolysates, lactose and xylose or a solution of hemicellulolytic hydrolysates, the quantities of each of the constituents of the mixture being defined by the following limits:
• 40% to 65% by weight of glucose;
• 21 % to 25% by weight of lactose; and
• 10% to 39% by weight of xylose;
the sum of these three constituents being equal to 100%, and the microorganism belongs to the species *Trichoderma reesei* and is deleted for catabolic repression by glucose.

2. A process according to claim 1 , in which the inducing substrate is supplied in solution, the concentration of inducing substrate in the supply solution used during the production phase being 350 to 600 g/L.

3. A process according to claim 2, in which the concentration is in the range 450 to 550 g/L.

4. A process according to any one of the preceding claims, in which the mixture constituting the inducing substrate comprises 50% to 65% by weight of glucose, 22% to 24% by weight of lactose and 15% to 25% by weight of xylose, the sum of the constituents being equal to 100%.

5. A process according to claim 4, in which the inducing substrate is a mixture constituted by 60% by weight of glucose, 23% by weight of lactose and 17% by weight of xylose.

6. A process according to one of the preceding claims, in which the carbonaceous substrate used during the growth phase is selected from glucose, xylose, lactose, residues obtained after ethanolic fermentation of monomeric sugars of the enzymatic hydrolysates of cellulosic biomass and/or an unrefined extract of hydrosoluble pentoses possibly derived from the pre-treatment of a cellulosic biomass.

7. A process according to one of the preceding claims, in which the pH is adjusted to between 3.5 and 6 and the temperature is in the range 20°C to 35°C.

8. A process according to one of the preceding claims, in which the flow rate for introduction of the supply solution is 30 to 45 mg per gram of cells per hour.

9. A process according to one of the preceding claims, in which the residual concentration of sugar in the culture medium during the production phase is less than 1 g/L, preferably less than 0.5 g/L and highly preferably less than 0.1 g/L.
